(19) **Europäisches Patentamt European Patent Office Office européen des brevets**

(11) **EP 1 596 781 B1**

(12) **FASCICULE DE BREVET EUROPEEN**

(45) Date de publication et mention
de la délivrance du brevet:
**07.10.2009 Bulletin 2009/41**

(21) Numéro de dépôt: **04710897.2**

(22) Date de dépôt: **13.02.2004**

(51) Int Cl.:
***A61F 5/01*** *(2006.01)*

(86) Numéro de dépôt international:
**PCT/FR2004/000343**

(87) Numéro de publication internationale:
**WO 2004/078077 (16.09.2004 Gazette 2004/38)**

(54) **DISPOSITIF DE CONTENTION ELASTIQUE DES CUISSES**

VORRICHTUNG ZUM ELASTISCHEN HALTEN DER OBERSCHENKEL

DEVICE FOR ELASTIC RESTRAINT OF THE THIGHS

(84) Etats contractants désignés:
**AT BE BG CH CY CZ DE DK EE ES FI FR GB GR HU IE IT LI LU MC NL PT RO SE SI SK TR**
Etats d'extension désignés:
**LT LV**

(30) Priorité: **21.02.2003 FR 0302194**

(43) Date de publication de la demande:
**23.11.2005 Bulletin 2005/47**

(73) Titulaire: **Nicolas, Philippe**
**35470 Bain de Bretagne (FR)**

(72) Inventeur: **Nicolas, Philippe**
**35470 Bain de Bretagne (FR)**

(74) Mandataire: **Bomer, Françoise Marie**
**Cabinet Regimbeau**
**Espace Performance**
**Bâtiment K**
**35769 Saint-Grégoire-Cedex (FR)**

(56) Documents cités:
| | |
|---|---|
| **WO-A-99/03541** | **WO-A-99/45802** |
| **US-A- 5 267 928** | **US-A- 5 286 251** |
| **US-A- 5 718 672** | |

## Description

La présente invention concerne un dispositif de contention élastique destiné à assurer un mouvement d'adduction des cuisses d'un être humain dit "utilisateur", portant ce dispositif.

Il trouve une application particulière dans la prévention ou la guérison des tendinites des muscles adducteurs de la cuisse.

Une tendinite est une inflammation d'un tendon utilisé de manière excessive, cette inflammation étant favorisée par une mauvaise posture.

Certains sportifs, et notamment ceux pratiquant la course à pied, le tennis, le football ou le rugby ou tout autre sport où l'on court beaucoup, souffrent plus particulièrement de tendinites des muscles situés sur la face interne des cuisses et dénommés "muscles adducteurs".

Le traitement thérapeutique classique de ce type de tendinite consiste à prescrire au sportif malade, des médicaments anti-inflammatoires, des séances de kinésithérapie à visée sédative et l'arrêt temporaire de l'entraînement sportif.

Un traitement thérapeutique complémentaire utilise l'acupuncture, la manipulations des muscles par ostéopathie et/ou le port de semelles orthopédiques.

L'inconvénient principal de cette façon de traiter les tendinites est sa durée importante et le risque de récidive. En outre, l'arrêt de l'entraînement chez un sportif de haut niveau peut être préjudiciable à sa carrière sportive.

Par ailleurs, dans le but de d'améliorer un mouvement sportif et de prévenir l'apparition des tendinites des muscles adducteurs qui peuvent survenir suite à la répétition de ce mouvement, les sportifs doivent essayer de corriger en permanence tout mouvement sportif imparfait, ce qui est extrêmement contraignant.

Le document US 5 718 672 A décrit un dispositif conforme au préambule de la revendication 1.

La présente invention a pour but de remédier aux inconvénients précités de l'état de la technique et de fournir un dispositif permettant de soigner et/ou de prévenir les tendinites des muscles adducteurs des cuisses tout en autorisant la poursuite d'un exercice physique minimum pour l'entretien des muscles.

Ce but est atteint à l'aide un dispositif de contention élastique permettant d'assurer un mouvement d'adduction des cuisses d'un être humain, dit "utilisateur" portant ce dispositif.

Conformément à l'invention, ce dispositif comprend une ceinture élastique destiné à enserrer la taille dudit utilisateur, deux sangles élastiques, dites "sangle droite" et "sangle gauche", destinées à enserrer respectivement la zone médiane des cuisses droite et gauche dudit utilisateur, et quatre bandes élastiques de contention, les première bande et deuxième bande de contention reliant un point de la sangle gauche, dit "point inférieur gauche", situé en regard de la zone médiane externe de la cuisse gauche de l'utilisateur, à un point de la ceinture élastique, dit "point supérieur droit", situé en regard du flanc droit de la taille de l'utilisateur, les troisième bande et quatrième bande de contention reliant un point de la sangle droite, dit "point inférieur droit", situé en regard de la zone médiane externe de la cuisse droite de l'utilisateur, à un point de la ceinture, dit "point supérieur gauche", situé en regard du flanc gauche de la taille de l'utilisateur, la première bande et la troisième bande étant destinées à être placées sur le devant du corps de l'utilisateur tandis que la deuxième bande et la quatrième bande sont destinées à être placées sur l'arrière du corps de l'utilisateur, les quatre bandes élastiques de contention étant d'une longueur telle qu'elles sont sous tension lorsque le dispositif est porté par l'utilisateur, de façon à assurer un mouvement d'adduction des cuisses de celui-ci.

Selon d'autres caractéristiques avantageuses et non limitatives de l'invention, prises seules ou en combinaison :

- le coefficient d'allongement de chaque bande élastique de contention est compris entre 10 et 25 % environ ;
- la première bande et la troisième bande sont fixées entre elles au niveau de leur point de chevauchement et/ou la deuxième bande et la quatrième bande sont fixées entre elles au niveau de leur point de chevauchement ;
- la fixation au niveau du point de chevauchement est effectuée à l'aide d'une couture ;
- la fixation des extrémités des bandes de contention sur la ceinture élastique et sur les sangles droite ou gauche s'effectue à l'aide d'une couture ;
- les deux extrémités de la ceinture élastique sont munies de moyens d'accrochage complémentaires permettant d'ajuster la longueur de ladite ceinture aux dimensions de la taille de l'utilisateur ;
- les deux extrémités d'au moins l'une des sangles élastiques sont munies de moyens d'accrochage complémentaires permettant d'ajuster la longueur de ladite sangle aux dimensions de la cuisse de l'utilisateur.

D'autres caractéristiques et avantages de l'invention apparaîtront à la lecture de la description qui va maintenant en être faite, en référence aux dessins annexés, qui en représentent, à titre indicatif mais non limitatif, un mode de réalisation possible.

Sur ces dessins :

- la figure 1 est une vue schématique en perspective du dispositif de contention conforme à l'invention, disposé sur un utilisateur représenté de façon partielle,
- la figure 2 est une vue en perspective du dispositif de contention conforme à l'invention,
- les figures 3 et 4 sont des vues en coupe transversale de la taille et des cuisses de l'utilisateur, prises selon des plans de coupe horizontaux référencés respectivement P3 et P4 sur la figure 1, le côté gauche du corps étant situé à gauche sur ces figures, et

- la figure 5 représente de façon schématique la cuisse droite de l'utilisateur.

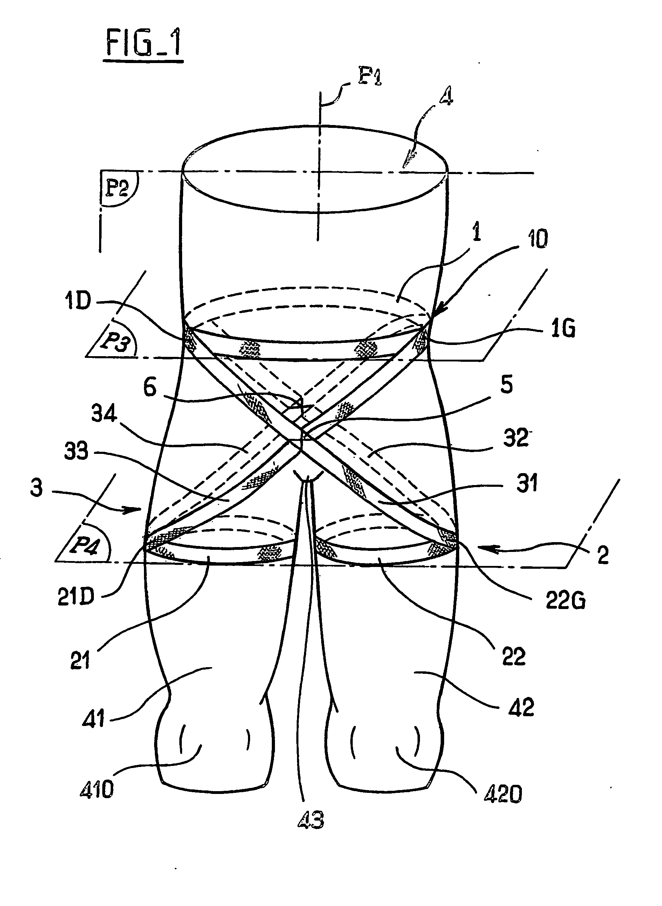

Le dispositif de contention élastique conforme à l'invention est destiné à être porté par un être humain, dit "utilisateur", représenté de façon partielle sur la figure 1 et portant la référence numérique générale 4.

L'utilisateur 4 présente une taille référencée 40, une cuisse 41 et un genou 410 droits, une cuisse 42 et un genou 420 gauches et un entrejambe référencé 43.

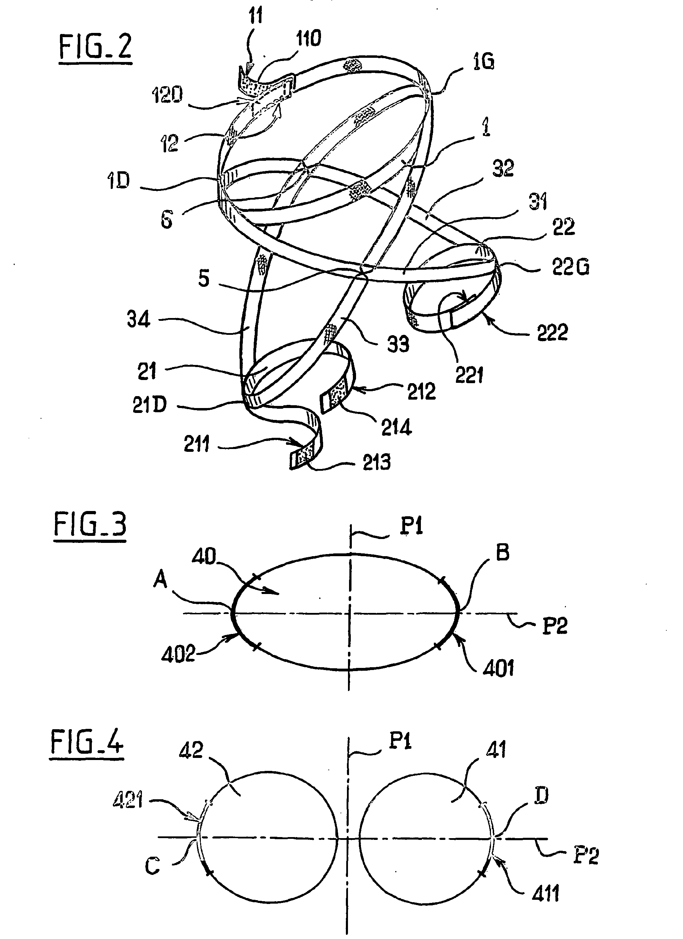

L'utilisateur 4 présente en outre un plan vertical médian de symétrie **P1,** représenté sur les figures 3 et 4, qui partage son corps en deux moitiés droite et gauche.

L'utilisateur 4 présente également un plan vertical médian **P2,** perpendiculaire au plan **P1** et qui partage son corps en une moitié avant et une moitié arrière.

Comme cela apparaît mieux sur la figure 3, l'utilisateur présente en outre au niveau de sa taille 40, deux zones latérales dites "flanc droit" 401 et "flanc gauche" 402.

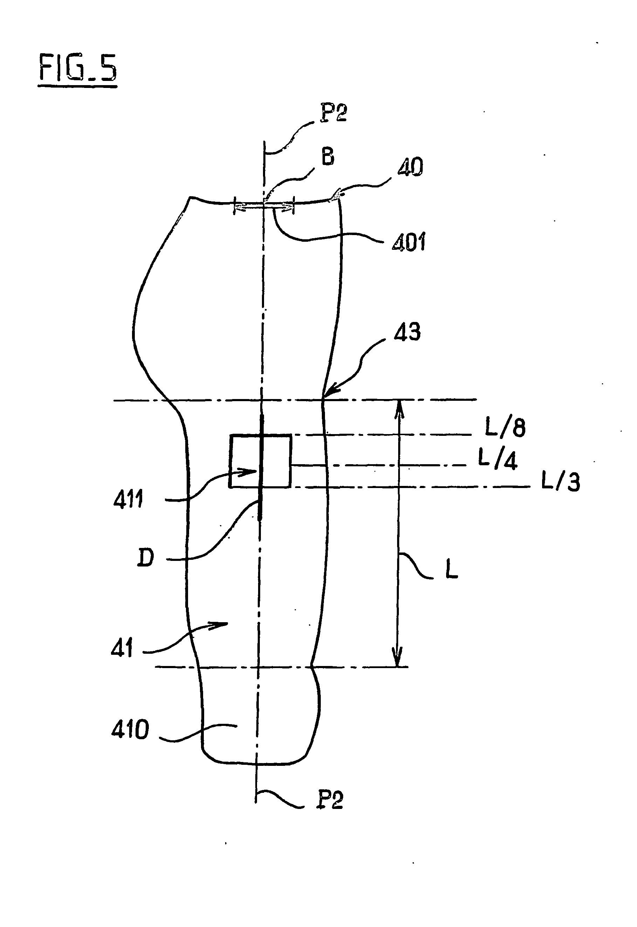

Dans la suite de la description et des revendications, le "flanc droit" 401 de la taille 40 est défini comme une zone en arc de cercle s'étendant de part et d'autre du point d'intersection **B** entre le plan vertical médian **P2** et le côté gauche de la circonférence de la taille 40, sur une faible distance vers l'avant et vers l'arrière du corps par rapport à ce point **B** (voir les figures 3 et 5).

Le "flanc gauche" 402 de la taille 40 est défini de façon similaire par rapport au point d'intersection A entre la plan vertical **P2** et le côté gauche de la circonférence de la taille 40.

En se reportant maintenant à la figure 4, on définit également deux zones dites "zone médiane externe" 421 de la cuisse gauche 42 et "zone médiane externe" 411 de la cuisse droite 41.

Dans la suite de la description et des revendications et comme cela apparaît sur les figures 4 et 5, la zone médiane externe 411 de la cuisse droite 41 est définie comme une zone en arc de cercle s'étendant de part et d'autre de la ligne d'intersection **D** entre le plan vertical médian **P2** et la face externe de la cuisse droite 41, cette zone 411 en arc de cercle s'étendant sur une faible distance vers l'avant et vers l'arrière du corps par rapport à cette ligne **D** et sur une hauteur comprise entre le huitième supérieur **L/8** et le tiers supérieur **L/3** de la longueur **L** de la cuisse 41 prise de l'entrejambe 43 à la partie supérieure du genou 410. Cette hauteur peut également être définie comme une zone s'étendant entre 10 et 15 centimètres (10 à 15 cm) sous le grand trochanter. De préférence, la zone 411 s'étend au voisinage du quart supérieur **L/4** de la longueur **L** de la cuisse.

La zone médiane externe 421 de la cuisse gauche 42 est définie de façon similaire par rapport à la ligne d'intersection **C** entre le plan **P2** et la face externe de la cuisse gauche 42.

Le dispositif de contention conforme à l'invention va maintenant être décrit en faisant référence aux figures 1 et 2.

Il se compose d'une ceinture élastique 1 destinée à enserrer la taille 40 de l'utilisateur 4, de deux sangles élastiques portant la référence générale 2 et de quatre bandes élastiques de contention portant la référence générale 3.

De préférence, tous ces éléments sont réalisés à partir d'une bande de tissu élastique d'une largeur de 3 à 4 cm environ. A titre d'exemple, on peut utiliser une bande de tissu élastique s'allongeant de 2 centimètres (2 cm) pour une bande d'un mètre étirée par un poids de 1 Kg. Il serait également possible d'utiliser des bandes de caoutchouc ou d'une matière plastique présentant une certaine élasticité.

La ceinture élastique 1 peut être constituée d'une bande de matériau élastique refermée sur elle-même en boucle et dont les deux extrémités sont cousues ou collées. Sa longueur est alors calculée pour être très légèrement inférieure à la circonférence de la taille 40 de l'utilisateur 4, de façon à enserrer celle-ci suffisamment pour que la ceinture 1 ne glisse pas vers le bas et reste en place au niveau de la taille, mais sans entraver les mouvements ou la respiration.

Selon une variante de réalisation illustrée sur la figure 2, les deux extrémités 11, 12 de la ceinture élastique 1 sont munies de moyens d'accrochage complémentaires 110, 120 permettant d'ajuster la longueur de ladite ceinture 1 aux dimensions de la taille 40 de l'utilisateur 4, de façon à pouvoir fixer cette ceinture 1 comme mentionné ci-dessus. Ces moyens complémentaires d'accrochage 110, 120 permettent d'adapter le dispositif conforme à l'invention à des utilisateurs de corpulence différente.

Ces moyens d'accrochage 110, 120 peuvent être une boucle de ceinture fixée sur l'une des extrémités de la ceinture 1 et des perforations prévues dans l'autre extrémité de cette ceinture.

De préférence, ces moyens d'accrochage 110, 120 sont des bandes de tissu à crochets et bouclettes, connues sous la marque déposée "Velcro".

Les sangles élastiques 2 comprennent une sangle droite 21 et une sangle gauche 22, destinées à enserrer respectivement la cuisse droite 41 et la cuisse gauche 42 de l'utilisateur 4.

La sangle droite 21 doit être placée à une hauteur située en regard de la zone médiane externe 411 de la cuisse droite 41 telle que définie précédemment et de préférence, au voisinage du tiers supérieur **L/3** de la cuisse droite 41.

La sangle gauche 22 doit être disposée de façon similaire sur la cuisse gauche 42 de l'utilisateur 4.

Chaque sangle doit enserrer suffisamment la cuisse autour de laquelle elle est placée pour rester dans la position définie ci-dessus mais sans toutefois gêner le mouvement ou entraver la circulation sanguine.

De façon similaire à ce qui a été décrit précédemment pour la ceinture 1, les sangles 21 ou 22 peuvent être constituées d'une bande de tissu élastique dont les deux extrémités sont fixées l'une à l'autre, par exemple par couture voire collage, ou dont les deux extrémités 211, 212, respectivement 221, 222 sont munies de moyens complémentaires d'accrochage.

Ces moyens complémentaires d'accrochage, représentés uniquement sur la sangle droite 21 sont de préférence des bandes 213, 214 de tissu à crochets et bouclettes, connues sous la marque déposée "Velcro" et rendues solidaires des faces des extrémité 211, 212 de la sangle 21 destinée à se chevaucher.

Enfin, les première, deuxième, troisième et quatrième bandes de contention élastique portent respectivement les références numériques 31, 32, 33 et 34.

Comme cela apparaît mieux sur la figure 1, la première bande 31 et la deuxième bande 32 de contention relient un point de la sangle gauche 22, dit "point inférieur gauche" 22G, qui, lorsque le dispositif de contention est en place sur l'utilisateur 4, est situé en regard de la zone médiane externe 421 de la cuisse gauche 42 telle que définie précédemment, à un point de la ceinture élastique 1, dit "point supérieur droit" 1D, situé en regard du flanc droit 401 de la taille 40 tel que défini précédemment.

Inversement, la troisième bande 33 et la quatrième bande 34 de contention relient un point de la sangle droite 21, dit "point inférieur droit" 21D, qui lorsque le dispositif de contention est porté, est situé en regard de la zone médiane externe 411 de la cuisse droite 41 de l'utilisateur, à un point de la ceinture 1, dit "point supérieur gauche" 1G, situé en regard du flanc gauche 402 de la taille 40 de l'utilisateur.

La position mentionnée ci-dessus pour les points de fixation 1D, 1 G, 21D et 21G est importante pour obtenir l'effet d'adduction recherché, assuré par le dispositif de contention conforme à l'invention.

A leurs deux extrémités, chacune des quatre bandes de contention élastique 31, 32, 33 et 34 est fixée sur la ceinture 1 et sur les sangles 21 ou 22 de préférence à l'aide d'une couture. Elles pourraient également y être collées.

De préférence encore, les point 1D, 1G, 21D et 21G précités sont situés dans le plan vertical médian **P2.**

La première bande 31 et la troisième bande 33 se croisent en passant sur le devant du corps de l'utilisateur, tandis que la deuxième bande 32 et la quatrième bande 34 se croisent sur l'arrière du corps de l'utilisateur sensiblement au niveau des fesses de ce dernier.

La longueur de ces quatre bandes élastiques de contention 31, 32, 33 et 34 est telle que ces dernières sont sous tension lorsque le dispositif de contention est porté par l'utilisateur.

On définit le coefficient d'allongement **CA** d'une bande élastique donnée selon la formule suivante :

$$CA = [(L1 - L0) / L0] \times 100$$

**CA** étant exprimé en pourcentage, **L0** représentant la longueur "à vide" de ladite bande, c'est-à-dire lorsque le dispositif de contention n'est pas porté et **L1** la longueur de cette même bande lorsque le dispositif de contention est porté par l'utilisateur.

Le coefficient d'allongement **CA** des bandes de contention 31, 32, 33 est de préférence compris entre 10 et 25 % environ et de préférence encore voisin de 20 %. A titre d'exemple, si des bandes de contention 31, 32 ayant un coefficient d'allongement de 20 % présentent une longueur **L0** d'environ 45 cm, leur longueur **L1** atteint environ 54 cm lorsque le dispositif de contention est porté par l'utilisateur.

On notera que le coefficient d'allongement CA de la ceinture 1 et des sangles 21 et 22 est de l'ordre de 5 %.

De façon avantageuse, la première bande 31 et la troisième bande 33 sont fixées entre elles au niveau de leur point de chevauchement 5, de préférence à l'aide d'une couture. Les deuxième et quatrième bandes 32, 34 sont fixées au niveau de leur point de chevauchement 6 de façon similaire.

On notera que selon une variante de réalisation, la première et la deuxième bande de contention 31, 32 pourraient être remplacées par une unique bande de matériau élastique pliée en deux. Il en est de même pour les troisième et quatrième bandes de contention 33, 34.

Le dispositif de contention conforme à l'invention permet d'exercer un mouvement d'adduction sur les cuisses 41, 42 de l'utilisateur, notamment grâce à la tension des quatre bandes élastiques de contention 31, 32, 33 et 34 et aux forces qu'elles exercent, c'est-à-dire que les cuisses 41 et 42 ont tendance à être rapprochées du plan vertical médian de symétrie **P1** du corps de l'utilisateur.

Bien entendu, les dimensions de la ceinture 1, des sangles 21 et 22 et des quatre bandes de contention sont adaptées à la morphologie de l'utilisateur pour obtenir l'effet d'adduction recherché.

Le dispositif conforme à l'invention permet de soigner les tendinites des muscles adducteurs des cuisses en réalisant le mouvement d'adduction des cuisses en direction du plan vertical **P1** sans solliciter les muscles adducteurs qui de ce fait peuvent rester au repos.

Ce dispositif permet également de corriger le geste sportif de la course, en obligeant le porteur de ce dispositif à courir avec l'axe longitudinal des cuisses maintenu dans un plan sensiblement vertical.

## Revendications

1. Dispositif de contention élastique permettant d'assurer un mouvement d'adduction des cuisses d'un être humain dit "utilisateur" (4) le portant, ce dispositif comprenant deux sangles élastiques dites "sangle droite" (21) et "sangle gauche" (22), destinées à enserrer respectivement la zone médiane des cuisses droite (41) et gauche (42) dudit utilisateur (4), ce dispositif étant **caractérisé en ce qu'**il comprend une ceinture élastique (1) destinée à enserrer la taille (40) dudit utilisateur (4) et quatre bandes élastiques de contention (31, 32, 33, 34), les première bande (31) et deuxième bande (32) de contention reliant un point de la sangle gauche (22), dit "point inférieur gauche" (22G), situé en regard de la zone médiane externe (421) de la cuisse gauche (42) de l'utilisateur (4), à un point de la ceinture élastique (1), dit "point supérieur droit" (1D), situé en regard du flanc droit (401) de la taille (40) de l'utilisateur (4), les troisième bande (33) et quatrième bande (34) de contention reliant un point de la sangle droite (21), dit "point inférieur droit" (21D), situé en regard de la zone médiane externe (411) de la cuisse droite (41) de l'utilisateur (4), à un point de la ceinture (1), dit "point supérieur gauche" (1G), situé en regard du flanc gauche (402) de la taille (40) de l'utilisateur (4), la première bande (31) et la troisième bande (33) étant destinées à être placées sur le devant du corps de l'utilisateur tandis que la deuxième bande (32) et la quatrième bande (34) sont destinées à être placées sur l'arrière du corps de l'utilisateur, les quatre bandes élastiques de contention (31, 32, 33, 34) étant d'une longueur telle qu'elles sont sous tension lorsque le dispositif est porté par l'utilisateur (4), de façon à assurer un mouvement d'adduction des cuisses (41, 42) de celui-ci.

2. Dispositif selon la revendication 1, **caractérisé en ce que** le coefficient d'allongement de chaque bande élastique de contention (31, 32, 33, 34) est compris entre 10 et 25 % environ.

3. Dispositif selon la revendication 1 ou 2, **caractérisé en ce que** la première bande (31) et la troisième bande (33) sont fixées entre elles au niveau de leur point de chevauchement (5) et/ou **en ce que** la deuxième bande (32) et la quatrième bande (34) sont fixées entre elles au niveau de leur point de chevauchement (6).

4. Dispositif selon la revendication 3, **caractérisé en ce que** la fixation au niveau du point de chevauchement (5,6) est effectuée à l'aide d'une couture.

5. Dispositif selon l'une quelconque des revendications précédentes, **caractérisé en ce que** la fixation des extrémités des bandes de contention (31, 32, 33, 34) sur la ceinture élastique (1) et sur les sangles droite (21) ou gauche (22) s'effectue à l'aide d'une couture.

6. Dispositif selon l'une quelconque des revendications précédentes, **caractérisé en ce que** les deux extrémités (11, 12) de la ceinture élastique (1) sont munies de moyens d'accrochage complémentaires (110, 120) permettant d'ajuster la longueur de ladite ceinture (1) aux dimensions de la taille (40) de l'utilisateur (4).

7. Dispositif selon l'une quelconque des revendications précédentes, **caractérisé en ce que** les deux extrémités (211, 212, 221, 222) d'au moins l'une des sangles élastiques (21, 22) sont munies de moyens d'accrochage complémentaires (213, 214) permettant d'ajuster la longueur de ladite sangle (21, 22) aux dimensions de la cuisse (41, 42) de l'utilisateur (4).

**Claims**

1. Elastic restraint device that allows an adduction movement of the thighs of a human being, referred to as the "user" (4) wearing it, which device includes two elastic straps, referred to as a "right strap" (21) and a "left strap" (22), intended to respectively hold the median area of the right (41) and left (42) thighs of the user (4), which device is **characterised in that** it includes an elastic belt (1) intended to hold the waste (40) of said user (4) and four elastic restraint bands (31, 32, 33, 34), wherein the first restraint band (31) and the second restraint band (32) connect a point of the left strap (22), referred to as the "lower left point" (22G), located opposite the outer median area (421) of the left thigh (42) of the user (4) to a point on the elastic belt (1), referred to as the "upper right point" (1D), located opposite the right flank (401) of the waist (40) of the user (4), and the third constraint band (33) and the fourth constraint band (34) connect a point on the right strap (21), referred to as the "lower right point" (21D), located opposite the outer median area (411) of the right thigh (41) of the user (4), to a point on the belt (1), referred to as the "upper left point" (1G), located opposite the left flank (402) of the waist (40) of the user (4), and the first band (31) and the third band (33) are intended to be placed on the front of the user's body while the second band (32) and the fourth band (34) are intended to be placed on the back of the user's body, and the four elastic restraint bands (31, 32, 33, 34) have a length so that they are under tension when the device is worn by the user (4), so as to ensure an adduction movement of the thighs (41, 42) of said user.

2. Device according to claim 1, **characterised in that** the elongation coefficient of each elastic restraint band (31, 32, 33, 34) is between around 10 and 25%.

3. Device according to claim 1 or 2, **characterised in**

**that** the first band (31) and the third band (33) are attached to one another at the level of their point of overlap (5) and/or **in that** the second band (32) and the fourth band (34) are attached to one another at the level of their point of overlap (6).

4. Device according to claim 3, **characterised in that** the attachment at the level of the point of overlap (5, 6) is achieved by sewing.

5. Device according to any one of the previous claims, **characterised in that** the attachment of the ends of the restraint bands (31, 32, 33, 34) to the elastic belt (1) and to the right strap (21) or the left strap (22) is achieved by sewing.

6. Device according to any one of the previous claims, **characterised in that** the two ends (11, 12) of the elastic belt (1) are equipped with complementary hooking means (110, 120) making it possible to adjust the length of said belt (1) to the size of the waist (40) of the user (4).

7. Device according to any one of the previous claims, **characterised in that** the two ends (211, 212, 221, 222) of at least one of the elastic straps (21, 22) are equipped with complementary hooking means (213, 214) making it possible to adjust the length of said strap (21, 22) to the size of the thigh (41, 42) of the user (4).

**Patentansprüche**

1. Elastische Haltevorrichtung, welche erlaubt, eine Adduktionsbewegung der Oberschenkel eines Menschen, "Benutzer" (4) genannt, der sie trägt, zu gewährleisten, wobei diese Vorrichtung zwei elastische Gurte, "rechter Gurt" (21) und "linker Gurt" (22) genannt, umfasst, die dazu bestimmt sind, jeweils den mittleren Bereich des rechten (41) und linken (42) Oberschenkels des Benutzers (4) zu umschließen, wobei diese Vorrichtung **dadurch gekennzeichnet ist, dass** sie einen elastischen Gürtel (1) umfasst, der dazu bestimmt ist, die Taille (40) des Benutzers (4) zu umschließen, sowie vier elastische Haltebänder (31, 32, 33, 34), wobei das erste Halteband (31) und das zweite Halteband (32) einen Punkt des linken Gurts (22), "unterer linker Punkt" (22G) genannt, der gegenüber dem äußeren mittleren Bereich (421) des linken Oberschenkels (42) des Benutzers (4) liegt, mit einem Punkt des elastischen Gürtels (1), "oberer rechter Punkt" (1D) genannt, der gegenüber der rechten Seite (401) der Taille (40) des Benutzers (4) liegt, verbindet, wobei das dritte Halteband (33) und das vierte Halteband (34) einen Punkt des rechten Gurts (21), "unterer rechter Punkt" (21D) genannt, der gegenüber dem äußeren mittleren Bereich (411) des rechten Oberschenkels (41) des Benutzers (4) liegt, mit einem Punkt des Gürtels (1), "oberer linker Punkt" (1G) genannt, der gegenüber der linken Seite (402) der Taille (40) des Benutzers (4) liegt, verbinden, wobei das erste Band (31) und das dritte Band (33) dazu bestimmt sind, auf der Vorderseite des Körpers des Benutzers platziert zu werden, während das zweite Band (32) und das vierte Band (34) dazu bestimmt sind, auf der Rückseite des Körpers des Benutzers platziert zu werden, wobei die vier elastischen Haltebänder (31, 32, 33, 34) von einer solchen Länge sind, dass sie unter Spannung sind, wenn die Vorrichtung vom Benutzer (4) getragen wird, um eine Adduktionsbewegung seiner Oberschenkel (41, 42) zu gewährleisten.

2. Vorrichtung gemäß Anspruch 1, **dadurch gekennzeichnet, dass** der Dehnungskoeffizient jedes elastischen Haltebands (31, 32, 33, 34) ungefähr zwischen 10 und 25% ist.

3. Vorrichtung gemäß Anspruch 1 oder 2, **dadurch gekennzeichnet, dass** das erste Band (31) und das dritte Band (33) aneinander auf Höhe ihres Überlappungspunkts (5) befestigt sind und/oder das zweite Band (32) und das vierte Band (34) aneinander auf Höhe ihres Überlappungspunkts (6) befestigt sind.

4. Vorrichtung gemäß Anspruch 3, **dadurch gekennzeichnet, dass** die Befestigung auf Höhe des Überlappungspunkts (5,6) mittels einer Naht gemacht wird.

5. Vorrichtung gemäß einem der vorhergehenden Ansprüche, **dadurch gekennzeichnet, dass** die Befestigung der Enden der Haltebänder (31, 32, 33, 34) auf dem elastischen Gürtel (1) und auf dem rechten (21) oder linken (22) Gurt mittels einer Naht gemacht wird.

6. Vorrichtung gemäß einem der vorhergehenden Ansprüche, **dadurch gekennzeichnet, dass** die beiden Enden (11, 12) des elastischen Gürtels (1) mit komplementären Kopplungsmitteln (110, 120) ausgestattet sind, die erlauben, die Länge des Gürtels (1) den Abmessungen der Taille (40) des Benutzers (4) anzupassen.

7. Vorrichtung gemäß einem der vorhergehenden Ansprüche, **dadurch gekennzeichnet, dass** die beiden Enden (211, 212, 221, 222) des wenigstens einen der elastischen Gurte (21, 22) mit komplementären Kopplungsmitteln (213, 214) ausgestattet sind, die erlauben, die Länge des Gurts (21, 22) an die Abmessungen des Oberschenkels (41, 42) des Benutzers (4) anzupassen.

# FIG_1

FIG. 2

FIG. 3

FIG. 4

# FIG.5

**EP 1 596 781 B1**

## RÉFÉRENCES CITÉES DANS LA DESCRIPTION

**Documents brevets cités dans la description**

- US 5718672 A